# EUROPÄISCHE PATENTSCHRIFT

(11) **EP 0 491 142 B1**
(45) Veröffentlichungstag und Bekanntmachung des Hinweises auf die Patenterteilung: **26.05.1993**
(21) Anmeldenummer: 91118213.7
(22) Anmeldetag: 25.10.1991
(51) Int. Cl.: C07D 317/20, C07D 319/06

(54) **Verfahren zur Herstellung von Gemischen cyclischer Acroleinglycerinacetale**
Process for the preparation of mixtures of cyclic glycerin acetals of acrolein
Procédé pour la préparation de mélanges d'acétals de glycérine cycliques d'acroléine

(30) Priorität: 17.12.1990 DE 4040362
(43) Veröffentlichungstag der Anmeldung: 24.06.1992
(73) Patentinhaber: Degussa Aktiengesellschaft, 60311 Frankfurt (DE)
(72) Erfinder: Höpp, Mathias, Dr., W-6465 Biebergemünd (DE); Arntz, Dietrich, Dr., W-6370 Oberursel (DE); Bartsch, Stephan, Dr., W-6463 Freigericht (DE); Schäfer-Sindlinger, Adolf, Dr., W-6000 Frankfurt 60 (DE); Böck, Wolfgang, Dr., W-6456 Langenselbold (DE)

(56) Entgegenhaltungen:
- US-A- 3 014 924
- US-A- 3 250 788
- US-A- 4 003 918

## Beschreibung

Die Erfindung betrifft ein Verfahren zur Herstellung von Gemischen cyclischer Acroleinglycerinacetale. Es handelt sich hierbei um Gemische aus cis-2-Vinyl-4-hydroxymethyl-1,3-dioxolan, trans-2-Vinyl-4-hydroxymethyl-1,3-dioxolan, cis-2-Vinyl-5-hydroxy-1,3-dioxan und trans-2-Vinyl-5-hydroxy-1,3-dioxan. Das Verfahren basiert auf der säurekatalysierten Umsetzung von Acrolein mit Glycerin und eignet sich insbesondere zur kontinuierlichen Herstellung von Gemischen cyclischer Acroleinglycerinacetale.

Es ist bekannt, cyclische Acetale des Acroleins mit Glycerin durch Umsetzung von Acrolein und Glycerin in Gegenwart eines festen sauren Katalysators herzustellen - US-PS 3,014,924 und J. Org. Chem. (1960), 319-324. Als Katalysator dienen mit Mineralsäuren belegte hochporöse Trägermaterialien, wie Kieselsäure, Kieselgel, Silicoaluminate. Die Umsetzung erfolgt bei Temperaturen zwischen 50 und 150 °C, insbesondere 100 und 125 °C, wobei mittels eines organischen Lösungsmittels, wie beispielsweise Benzol, Toluol, Chloroform oder Cyclohexan, das Reaktionswasser azeotrop abdestilliert wird. Nachteilig an diesem Verfahren ist die geringe Raum-Zeit-Ausbeute, welche unter anderem darauf zurückgeführt werden kann, daß die Hauptmenge des Glycerins zunächst als separate Phase vorliegt. Die bei Verwendung einer geringen Katalysatormenge erforderliche hohe Reaktionstemperatur birgt in Verbindung mit der langen Reaktionsdauer die Gefahr der Nebenproduktbildung; eine erhöhte Katalysatormenge beschleunigt zwar die Acetalbildung, gleichzeitig werden aber Nebenprodukte in nicht tolerierbarer Menge gebildet. Im Hinblick auf eine kontinuierliche Prozeßführung wird von der Verwendung von Acrolein als Azeotropschleppmittel wegen dadurch begünstigter Nebenproduktbildung abgeraten. Schließlich wird durch die Verwendung eines artfremden organischen Lösungsmittels der verfahrenstechnische Aufwand nicht unbeträchtlich erhöht und die Wirtschaftlichkeit gemindert. Die Nacharbeitung des Verfahrens der US-PS 3,014,924 im technischen Maßstab - siehe Vergleichsbeispiel - bestätigt die unbefriedigende Raum-Zeit-Ausbeute und macht deutlich, daß auf dieser Grundlage ein kontinuierliches Verfahren nicht wirtschaftlich betrieben werden kann.

Es ist auch bekannt, Acrolein unter homogener Katalyse mit einem 1,3-Diol umzusetzen, wobei Mineralsäuren oder Sulfonsäuren als Katalysator dienen (US-PS 4,108,869). Das Diol wird im Überschuß von oben in eine Extraktionssäule eingespeist und enthält die Säure. In der Mitte wird Acrolein eingespeist, welches mit dem nach unten fließenden Diol reagiert. Von unten wird im Gegenstrom ein Lösungsmittel eingespeist, welches sich mit dem Diol schlecht mischt, z. B. Hexan. Die Hexan- und die Diol/Wasser-Phase werden destillativ aufgearbeitet. Dieses Verfahren läßt sich nicht auf die Herstellung von Acroleinglycerinacetalen übertragen, weil sich Acrolein und Glycerin nicht miteinander mischen und daher in der Extraktionssäule keine ausreichende Umsetzung erfolgt.

In einem Vergleichsbeispiel der US-PS 4,108,869 wird ein stark saurer Kationenaustauscher als Katalysator für die Umsetzung von 2-Methyl-1,3-propandiol mit Acrolein vorgeschlagen. Das zunächst homogene Reaktionsgemisch trennt sich in eine Acetal- und eine wäßrige Phase. Wegen der Nichtmischbarkeit von Acrolein mit Glycerin kann das zuvor beschriebene Verfahren nicht zur wirtschaftlichen Herstellung der gewünschten cyclischen Acroleinglycerinacetalgemische herangezogen werden.

Aufgabe der vorliegenden Erfindung ist, ein Verfahren zur Herstellung von Gemischen cyclischer Acroleinglycerinacetale zur Verfügung zu stellen, das die Nachteile des aus der US-PS 3,014,924 bekannten Verfahrens nicht aufweist. Das Verfahren sollte eine hohe Raum-Zeit-Ausbeute bei hohen Selektivitäten erbringen und in Abwesenheit artfremder Lösungsmittel durchführbar sein. Schließlich sollte das Verfahren auch kontinuierlich in wirtschaftlicher Weise durchführbar sein.

Gefunden wurde ein Verfahren zur Herstellung von Gemischen cyclischer Acroleinglycerinacetale durch Umsetzung von Acrolein mit Glycerin in Gegenwart eines festen sauren Katalysators und destillative Aufarbeitung des Reaktionsgemisches, das dadurch gekennzeichnet ist, daß man die Umsetzung in Gegenwart des im wesentlichen aus Acrolein, Glycerin, cyclischen Acroleinglycerinacetalen und Wasser bestehenden Reaktionsgemischs als Lösungsmittel und in Abwesenheit artfremder Lösungsmittel durchführt und, soweit erforderlich, das vom Katalysator befreite Reaktionsgemisch vor der destillativen Aufarbeitung mit einer solchen Menge eines pH-Wert erhöhenden Stoffes versetzt, daß es in zehnfacher Verdünnung mit Wasser einen pH-Wert im Bereich von 4,5 bis 7 aufweist.

Die Unteransprüche richten sich auf bevorzugte Ausführungsformen des Verfahrens.

Das erfindungsgemäße Verfahren führt zu einer hohen Raum-Zeit-Ausbeute und Selektivität der Umsetzung. Überraschenderweise kann die Umsetzung bei niedrigen Temperaturen und ohne azeotrope Entwässerung durchgeführt werden. Somit bedarf es weder der aufwendigen Verwendung eines artfremden Lösungsmittels noch der selektivitätsmindernden und auch aus sicherheitstechnischer Sicht nicht unbedenklichen azeotropen Entwässerung mittels Acrolein.

Da Acrolein und Glycerin nicht miteinander mischbar sind, dient das gebildete Acroleinglycerinacetal als Lösevermittler. Das Reaktionsgemisch, das als Lösungsmittel eingesetzt wird, besteht im wesentlichen aus einem Gemisch cyclischer Acroleinglycerinacetale sowie den Reaktionspartnern Acrolein und Glycerin und dem Reaktionswasser. Unter dem Begriff "im wesentlichen" wird verstanden, daß das Reaktionsgemisch zusätzlich reaktionsbedingte Nebenprodukte enthalten kann. Die Zusammensetzung des Reaktionsgemischs richtet sich nach dem gewählten Molverhältnis Acrolein zu Glycerin und der Reaktionstemperatur und entspricht vorzugsweise der jeweiligen Gleichgewichtszusammensetzung. Das für das erfindungsgemäße Verfahren zunächst erforderliche Reaktionsgemisch kann in an sich beliebiger Weise hergestellt werden, etwa durch Mischen der Bestandteile. Prinzipiell können auch Glycerin und Acrolein in Abwesenheit eines Lösungsmittels zum benötigten Gleichgewichts-Reaktionsgemisch umgesetzt werden, wobei hier aber die Umsetzung zunächst im Zweiphasensystem erfolgt und somit eine lange Reaktionszeit erforderlich ist. Um bei der erfindungsgemäßen Acetalbildung eine Zweiphasenbildung zu vermeiden, sollte das Reaktionsgemisch stets eine ausreichende Menge Acroleinglycerinacetalgemisch, vorzugsweise 35 bis 50 Gew.-%, enthalten. Die geeignete Menge des als Lösungsmittel dienenden Reaktionsgemischs sowie die Konzentration an Acroleinglycerinacetalen läßt sich durch Orientierungsversuche leicht ermitteln. Bei einem Molverhältnis von Acrolein zu Glycerin von 2 zu 1 und einer Reaktionstemperatur von 20 °C sollte die Konzentration an Acroleinglycerinacetalen im Reaktionsgemisch 36 Gew.-% nicht unterschreiten.

Das Molverhältnis Acrolein zu Glycerin ist für die Umsetzung wenig kritisch, und jede der beiden Ausgangskomponenten kann im Überschuß eingesetzt werden. Das Molverhältnis richtet sich zweckmäßigerweise nach der Aufarbeitung des Reaktionsgemischs: Soll ein reines Acetalgemisch gewonnen werden oder ein von Acrolein befreites Reaktionsgemisch mit niedrigem Glyceringehalt selbst verwertet werden können, ist ein Molverhältnis von Acrolein zu Glycerin von größer 1 zu 1 bis 5 zu 1 empfehlenswert, ein Molverhältnis im Bereich von 1,5 zu 1 bis 2,5 zu 1 wird dabei aber bevorzugt. Ein Überschuß an Acrolein gegenüber Glycerin erniedrigt ferner die Viskosität des Reaktionsgemischs. Die Reaktanden werden im gewünschten Molverhältnis zu dem als Lösungsmittel dienenden Reaktionsgemisch in der Weise zugegeben und darin verteilt, daß stets nur eine flüssige Phase neben der festen Katalysatorphase vorhanden ist.

Das erfindungsgemäße Verfahren kann sowohl bei niedriger Temperatur, aber auch Temperaturen bis 100 °C durchgeführt werden. Im allgemeinen wird man aber von dem erfindungsgemäß möglichen Vorteil niedriger Temperaturen Gebrauch machen und die Umsetzung bei 0 bis 50 °C, vorzugsweise 10 bis 30 °C und insbesondere 10 bis 20 °C, durchführen. Bei höherer Temperatur wird das Gleichgewicht mehr zur Seite der Einsatzstoffe verschoben und zudem werden vermehrt Nebenprodukte gebildet; bei sehr niedriger Temperatur ist die Reaktionsgeschwindigkeit ggf. zu gering, um die angestrebte hohe Raum-Zeit-Ausbeute zu erzielen, und zudem kann die bei niedriger Temperatur gegebene erhöhte Viskosität des Reaktionsgemisch zu verfahrenstechnischen Problemen führen, etwa einem hohen Druckabfall, wenn das Reaktionsgemisch über einen Festbettkatalysator gepumpt wird. Üblicherweise erfolgt die Umsetzung bei Normaldruck; soweit erwünscht oder bei höherer Temperatur erforderlich, kann auch ein erhöhter Druck eingestellt werden.

Als Katalysator werden feste, im Reaktionsgemisch unlösliche saure Katalysatoren verwendet. Einsetzbar sind insbesondere anorganische und organische Ionenaustauscher in der H-Form, worunter verstanden wird, daß nicht alle austauschbaren Gruppen in der H-Form vorliegen müssen, sondern nur eine zweckentsprechende Anzahl, welche sich durch einen orientierenden Versuch ermitteln läßt. Stark saure Ionenaustauscher werden bevorzugt. Verwendbar sind auch anorganische Trägermaterialien mit hoher Oberfläche, welche eine starke Mineralsäure ausreichend fest adsorbiert enthalten - die Säure darf sich während der Umsetzung praktisch nicht von der Katalysatoroberfläche ablösen.

Unter den organischen Ionenaustauschern sind Austauscherharze auf der Basis von Styrol/Divinylbenzol-Copolymeren mit Sulfonat- oder Phosphonatgruppen, wobei solche mit stärker sauren Sulfonatgruppen bevorzugt werden, besonders geeignet; zweckmäßig ist es, makroporöse Ionenaustauscher zu verwenden. Auch marktübliche perfluorierte Sulfonsäureharze sind einsetzbar. Schwach saure Ionenaustauscher mit Carboxylgruppen erwiesen sich als kaum wirksam.

Unter den sauren anorganischen Ionenaustauschern werden bevorzugt solche auf der Basis polymerer Organosiloxane mit Sulfonatgruppen gemäß DE-PS 35 18 881 und DE-PS 32 26 093 verstanden. Einsetzbar sind ferner saure Zeolithe, deren SiO₂/Al₂O₃ größer 2 zu 1 ist, wie Zeolithe vom Typ Y, Mordenit, ZSM 5 und Silikalit, sowie Schichtsilikate mit sauren Zwischenschichten, etwa mit Mineralsäuren vorbehandelte Montmorillonite, verstanden.

Das erfindungsgemäße Verfahren läßt sich diskontinuierlich, beispielsweise in einem Rührreaktor, oder kontinuierlich, beispielsweise in einem Schlaufenreaktor, wobei der Katalysator als Festbett oder Fließbett angeordnet sein kann, durchführen. Prinzipiell sind alle Reaktorformen geeignet, welche einen ausreichenden Kontakt zwischen dem Reaktionsgemisch und dem festen Katalysator gewährleisten. Bei kontinuierlicher Verfahrensweise wird kontinuierlich Acrolein und Glycerin im gewünschten Molverhältnis zum Reaktionsgemisch eingespeist und die entsprechende Menge Reaktionsgemisch zur Aufarbeitung abgezogen. Für die Kontaktzeit des Reaktionsgemischs, ausgedrückt durch den LHSV-Wert (liquid hourly space velocity), gilt, daß große Verweilzeiten am Katalysator vermehrt Nebenprodukte liefern, geringe Verweilzeiten jedoch nicht genügend Umsatz liefern. Der LHSV-Wert (Liter Reaktionsgemisch pro Liter Katalysator (trocken) und Stunde) sollte zwischen 1 und 30 liegen, bevorzugt zwischen 3 und 20.

Bei kontinuierlicher Umsetzung im Rührkesselreaktor wird das Reaktionsgemisch zusammen mit dem Katalysator gerührt; bei kontinuierlicher Umsetzung im Schlaufenreaktor mit einem Katalysator-Festbett wird über dieses umgepumpt. Bei diskontinuierlicher Ausführung im Rührkessel werden die Reaktanden zu einer vorgelegten Menge Reaktionsgemisch gerade so schnell zugegeben, daß keine zweite Flüssigphase auftritt.

Das erfindungsgemäß gebildete Reaktionsgemisch weist vorzugsweise einen Gehalt von 35 bis 50 Gew.-%, insbesondere 40 bis 50 Gew.-%, an cyclischen Acroleinglycerinacetalen und reaktionsbedingt einen pH-Wert im Bereich von im allgemeinen 3,0 bis 6,0 auf, meist von 3 bis 4,5, jeweils gemessen in zehnfacher Verdünnung mit Wasser. Dem vom Katalysator befreiten Reaktionsgemisch muß, soweit erforderlich, vor der destillativen Aufarbeitung eine ausreichende Menge eines pH-Wert erhöhenden Stoffes zugegeben werden; eingestellt werden soll ein pH-Wert im Bereich von 4,5 bis 7, vorzugsweise 5,5 bis 7 und insbesondere 6 bis 7, gemessen in einer 10-fach mit Wasser verdünnten Probe. Als pH-Wert erhöhende Stoffe kommen gegenüber dem Reaktionsgemisch basisch wirkende Stoffe infrage; bevorzugt werden wäßrige Pufferlösungen mit einem pH-Wert im Bereich von 5 bis 7, insbesondere 5,5 bis 6,5, beispielsweise Citrat/NaOH-Puffer, Die pH-Erhöhung ist meist erforderlich, da sonst bei der Destillation des Acroleins Rückspaltung des Acetalgemischs, katalysiert durch die als Nebenprodukt gebildete Säure, eintritt. pH-Werte über 7 sind im Reaktionsgemisch zu vermeiden, da sonst spontane Polymerisation des Acroleins erfolgt. Im allgemeinen sind nur sehr kleine Mengen an pH-erhöhenden Stoffen, wie Pufferlösungen, erforderlich. Sofern der ph-Wert des aus dem Reaktor abgezogenen Reaktionsgemischs, gemessen in zehnfacher Verdünnung, um oder über 4,5 liegt, kann ggf. auf eine pH-Erhöhung vor der Destillation verzichtet werden.

Zur Aufarbeitung wird zunächst Acrolein abdestilliert, dann Wasser, dann die Acroleinglycerinacetale und zum Schluß das Glycerin. Das zurückgewonnene Acrolein und Glycerin werden erneut in den Reaktor eingespeist.

Es war nicht vorhersehbar, daß durch die Verwendung des Reaktionsgemisches als Lösungsmittel und die Neutralisation saurer Nebenprodukte vor der destillativen Aufarbeitung das Verfahren die bereits aufgezeigen Vorteile mit sich bringt. Die hohe Raum-Zeit-Ausbeute gestattet eine wirtschaftliche kontinuierliche Herstellung des Acetalgemischs. Die niedrige Reaktionstemperatur führt zu einer hohen Selektivität und damit reinen Produkten. Die Entbehrlichkeit eines artfremden Lösungsmittels und einer Azeotropdestillation führen zu einem geringen technischen Aufwand und niedrigen Herstellkosten.

### Vergleichsbeispiel

Das Beispiel 6 der US-PS 3,014,924 wurde in einen größeren Maßstab übertragen. Umgesetzt wurden 105 kg Acrolein, 139 kg Glycerin, 300 l Toluol in Gegenwart von 3,5 kg einer gefällten Kieselsäure belegt mit 1 % H₂SO₄. Bis zur vollständigen Wasserabscheidung ist eine Reaktionszeit von 9 Stunden erforderlich.

### Beispiel 1

In einen Schlaufenreaktor mit 40 l Gesamtvolumen ist ein Katalysatorfestbett mit 4 l Amberlyst® 15 (trocken) (ein stark saurer Ionenaustauscher der Fa. Rohm & Haas) eingebaut. Ein Reaktionsgemisch mit der ungefähren Zusammensetzung 32 Gew.-% Acrolein, 45 Gew.-% Aclroleinglycerinacetale, 12 Gew.-% Glycerin, 8 Gew.-% Wasser und 3 Gew.-% Nebenprodukte wird fortlaufend bei einer Innentemperatur von 15 °C im Kreis gepumpt. Kontinuierlich werden 16,2 kg Glycerin/h (176,0 Mol) und 19,8 kg Acrolein (96 %-ig)/h (339,4 Mol) eingespeist und die entsprechende Menge Reaktionsgemisch (36 kg/h) abgezogen (LHSV = 8,2). In das abfließende Reaktionsgemisch werden pro Liter kontinuierlich 2 ml eines 1-normalen Na-Citratpuffers mit pH 6 eingespeist. Das so erhaltende Reaktionsgemisch wird einer fraktionierten Destillation unterworfen, wobei stündlich 11,53 kg Acrolein (96 %-ig), 2,88 kg Wasser, 16,20 kg Acroleinglycerinacetale und 4,32 kg Glycerin gewonnen werden. Das zurückgewonnene Acrolein und Glycerin werden erneut eingesetzt. Das eingesetzte Acrolein wird zu 41,7 % umgesetzt mit einer Selektivität von 87,7 %, das eingesetzte Glycerin wird zu 73,3 % mit einer Selektivität von 96,5 % umgesetzt.

### Beispiel 2

In einen Rührkesselreaktor, beschickt mit 21 g Amberlyst® 15 und 320 ml Reaktionsgemisch der Zusammensetzung 33,5 % Acrolein, 11,1 % Glycerin, 43,9 % Acroleinglycerinacetal, 8,8 % Wasser und 2,7 % Nebenprodukte werden bei 15 °C Innentemperatur kontinuierlich 91 g Acrolein/h und 73 g Glycerin/h eingespeist. Die entsprechende Menge Reaktionsgemisch (164 g/h) (LHSV = 4,2) wird dem Reaktor über einen Überlauf fortlaufend entnommen und analysiert, wobei die Zusammensetzung weitgehend konstant bleibt. Das eingespeiste Acrolein wird zu 39,6 % umgesetzt mit einer Selektivität von 89,6 %. Das eingespeiste Glycerin wird zu 75 % umgesetzt mit einer Selektivität von 93,1 %.

### Beispiel 3

Wie Beispiel 2, jedoch mit einer gesteigerten Innentemperatur von 25 °C. Das erhaltene Reaktionsgemisch besitzt die Zusammensetzung 30,7 % Acrolein, 9,8 % Glycerin, 39,2 % Acroleinglycerinacetale, 8,2 % Wasser und 12,1 % Nebenprodukte. Acrolein wird zu 44,6 % mit einer Selektivität von 70,9 % und Glycerin zu 78,0 % mit einer Selektivität von 79,9 % umgesetzt.

### Beispiel 4

Wie Beispiel 2, jedoch mit niedrigeren Einspeisraten und somit erhöhten Verweilzeiten. Es werden 45,3 g Acrolein/h und 36,1 g Glycerin/h eingespeist. Das erhaltene Reaktionsgemisch besitzt die Zusammensetzung 31,5 % Acrolein, 6,7 % Glycerin, 43,4 % Acroleinglycerinacetale, 8,4 % Wasser und 10,0 % Nebenprodukte. Acrolein wird zu 43,4 % umgesetzt mit einer Selektivität von 77,4 %, Glycerin wird zu 84,8 % mit einer Selektivität von 81,7 % umgesetzt.

### Beispiel 5

Wie Beispiel 2, jedoch mit höheren Einspeisraten und somit niedrigeren Verweilzeiten (LHSV = 16,1). Es werden 357,0 g Acrolein/h und 264,6 g Glycerin/h eingespeist. Das erhaltene Reaktionsgemisch besitzt die Zusammensetzung 40,4 % Acrolein, 14,7 % Glycerin, 36,3 % Acroleinglycerinacetale, 7,1 % Wasser und 1,5 % Nebenprodukte. Acrolein wird zu 29,6 % umgesetzt mit einer Selektivität von 91,8 %, Glycerin wird zu 65,4 % mit einer Selektivität von 92,1 % umgesetzt.

### Beispiel 6

Wie Beispiel 2, jedoch mit verändertem Molverhältnis Acrolein zu Glycerin von 1 zu 1. Es werden 66,5 g Acrolein/h und 105,0 g Glycerin/h eingespeist. Das erhaltene Reaktionsgemisch besitzt die Zusammensetzung 14,5 % Acrolein, 22,1 % Glycerin, 46,0 % Acroleinglycerinacetal, 8,8 % Wasser und 8,6 % Nebenprodukte. Acrolein wird zu 62,6 % umgesetzt mit einer Selektiviät von 81,7 %, Glycerin wird zu 63,9 % mit einer Selektivität von 83,2 % umgesetzt.

### Beispiel 7

Wie Beispiel 2, jedoch mit verändertem Molverhältnis Acrolein zu Glycerin von 3 zu 1. Es werden 99,2 g Acrolein/h und 54,5 g Glycerin/h eingespeist. Das erhaltene Reaktionsgemisch besitzt die Zusammensetzung 45,8 % Acrolein, 4,7 % Glycerin, 37,6 % Acroleinglycerinacetal, 7,5 % Wasser und 4,4 % Nebenprodukte. Acrolein wird zu 29,0 % umgesetzt mit einer Selektiviät von 86,5 %, Glycerin wird zu 86,8 % mit einer Selektivität von 86,5 % umgesetzt.

### Beispiel 8

50 g Acrolein und 40 g Glycerin werden in einem Kolben vorgelegt. Unter Rühren werden 12 g Amberlyst® 15 zugegeben und die Innentemperatur wird auf 15 °C eingestellt. Sobald das Gemisch homogen ist, werden aus zwei Vorlagen weitere 286 g Acrolein und 236 g Glycerin über einen Zeitraum von 1 h gleichmäßig zugetropft, so daß das Reaktionsgemisch homogen bleibt. Nach beendeter Zugabe wird noch 30 Minuten nachgerührt, der Katalysator abfiltriert und das Gemisch mit 2 ml eines 1 N Na-Citratpuffers pH 6 versetzt. Die Analyse ergibt eine Zusammensetzung von 30,6 % Acrolein, 46,9 % Acroleinglycerinacetal, 10,9 % Glycerin, 9,2 Wasser und 2,4 % Nebenprodukte. Acrolein wird zu 44,1 % umgesetzt mit einer Selektivität von 83,5 %, Glycerin wird zu 75,9 % mit einer Selektivität von 96,6 % umgesetzt.

### Beispiel 9

Wie Beispiel 8 jedoch mit einem sulfonatgruppenhaltigen polymeren Organosiloxan (Typ ASP / Firma Degussa AG) als Katalysator:
50 g Acrolein und 40 g Glycerin werden in einem Kolben vorgelegt. Unter Rühren werden 12 g Polysiloxan ASP zugegeben und die Innentemperatur wird auf 15 °C eingestellt. Sobald das Gemisch homogen ist, werden aus zwei Vorlagen weitere 286 g Acrolein und 236 g Glycerin über einen Zeitraum von 3 h gleichmäßig zugetropft, so daß das Reaktionsgemisch homogen bleibt. Nach beendeter Zugabe wird noch 30 Minuten nachgerührt, der Katalysator abfiltriert und das Gemisch mit 2 ml eines 1 N Na-Citratpuffers pH 6 versetzt. Die Analyse ergibt eine Zusammensetzung von 34,3 % Acrolein, 39,9 % Acroleinglycerinacetal, 11,9 % Glycerin, 9,6 % Wasser und 4,3 % Nebenprodukte. Acrolein wird zu 37,5 % umgesetzt mit einer Selektivität von 83,3 %, Glycerin wird zu 73,6 % mit einer Selektivität von 85,0 umgesetzt.

## Patentansprüche

1. Verfahren zur Herstellung von Gemischen cyclischer Acroleinglycerinacetale durch Umsetzung von Acrolein mit Glycerin in Gegenwart eines festen sauren Katalysators und destillative Aufarbeitung des Reaktionsgemisches,
dadurch gekennzeichnet,
daß man die Umsetzung in Gegenwart des im wesentlichen aus Acrolein, Glycerin, cyclischen Acroleinglycerinacetalen und Wasser bestehenden Reaktionsgemischs als Lösungsmittel und in Abwesenheit artfremder Lösungsmittel durchführt und, soweit erforderlich, das vom Katalysator befreite Reaktionsgemisch vor der destillativen Aufarbeitung mit einer solchen Menge eines pH-Wert erhöhenden Stoffes versetzt, daß es in zehnfacher Verdünnung mit Wasser einen pH-Wert im Bereich von 4,5 bis 7 aufweist.

2. Verfahren nach Anspruch 1,
dadurch gekennzeichnet,
daß man die Umsetzung bei einer Temperatur im Bereich von 0 bis 50 °C, insbesondere 10 bis 30 °C, durchführt.

3. Verfahren nach den Ansprüchen 1 oder 2,
dadurch gekennzeichnet,
daß man als Katalysator stark saure organische oder anorganische Ionenaustauscher in der H-Form, insbesondere sulfonatgruppenhaltige Ionenaustauscher, verwendet.

4. Verfahren nach einem oder mehreren der Ansprüche 1 bis 3,
dadurch gekennzeichnet,
daß man Acrolein und Glycerin im Molverhältnis von größer 1 zu 1 bis 5 zu 1, insbesondere 1,5 bis 2,5 zu 1, einsetzt.

5. Verfahren nach einem oder mehreren der Ansprüche 1 bis 4,
dadurch gekennzeichnet,
daß man als Lösungsmittel ein Reaktionsgemisch einsetzt, das erhalten wurde aus der Umsetzung von Acrolein mit Glycerin im Molverhältnis von 1,5 bis 2,5 und 40 bis 50 Gew.-% des Gemischs an cyclischen Acroleinglycerinacetalen enthält.

6. Verfahren nach einem oder mehreren der Ansprüche 1 bis 5,
dadurch gekennzeichnet,
daß man als pH-Wert erhöhenden Stoff eine Pufferlösung mit einem pH-Wert im Bereich von 5 bis 7, vorzugsweise 5,5 bis 6,5, verwendet.

7. Verfahren nach Anspruch 6,
dadurch gekennzeichnet,
daß man das Reaktionsgemisch mit einer solchen Menge Pufferlösung versetzt, daß es in zehnfacher Verdünnung mit Wasser einen pH-Wert im Bereich von 5,5 bis 7 aufweist.

8. Verfahren nach einem oder mehreren der Ansprüche 1 bis 7,
dadurch gekennzeichnet,
daß man es kontinuierlich durchführt, indem man Acrolein und Glycerin im gewünschten Molverhältnis kontinuierlich zu dem als Lösungsmittel dienenden Reaktionsgemisch dosiert und in Gegenwart des festen sauren Katalysators umsetzt und die der Zudosierung entsprechende Menge Reaktionsgemisch fortlaufend entnimmt und der Aufarbeitung zuführt.

## Claims

1. A process for the preparation of mixtures of cyclic acrolein glycerol acetals by the reaction of acrolein with glycerol in the presence of a solid acid catalyst and distillative working up of the reaction mixture, characterised in that the reaction is carried out in the presence of the reaction mixture consisting substantially of acrolein, glycerol, cyclic acrolein glycerol acetals and water, which reaction mixture is used as solvent, and in the absence of any foreign solvents, and, if necessary, a substance which increases the pH is added in such quantity to the reaction mixture after the latter has been freed from catalyst and before it is worked up by distillation that the reaction mixture has a pH of from 4.5 to 7 when in tenfold dilution with water.

2. A process according to claim 1, characterised in that the reaction is carried out at a temperature in the range of from 0 to 50°C, in particular from 10 to 30°C.

3. A process according to claim 1 or claim 2, characterised in that the catalyst used is a strongly acid organic or inorganic ion exchanger in the H form, in particular sulphonate group-containing ion exchangers.

4. A process according to one or more of claims 1 to 3, characterised in that acrolein and glycerol are used in a molar ratio in the range of from > 1 : 1 to 5 : 1, in particular (1.5 - 2.5) : 1.

5. A process according to one or more of claims 1 to 4, characterised in that the solvent used is a reaction mixture obtained from the reaction of acrolein with glycerol in a molar ratio of from 1.5 to 2.5 and containing from 40 to 50% by weight of the mixture of cyclic acrolein glycerol acetals.

6. A process according to one or more of claims 1 to 5, characterised in that the pH increasing agent used is a buffer solution having a pH in the range of from 5 to 7, preferably from 5.5 to 6.5.

7. A process according to claim 6, characterised in that buffer solution is added to the reaction mixture in such a quantity than in tenfold dilution with water the reaction mixture has a pH in the range of from 5.5 to 7.

8. A process according to one or more of claims 1 to 7, characterised in that it is carried out continuously by continuously adding acrolein and glycerol in the desired molar ratio to the reaction mixture used as solvent and carrying out the reaction in the presence of the solid acid catalyst and continuously removing the quantity of reaction mixture corresponding to the quantity of acrolein and glycerol added and transferring it to the working up stage.

## Revendications

1. Procédé de préparation de mélange d'acroléineglycérineacétals en faisant réagir de l'acroléine avec la glycérine en présence d'un catalyseur acide solide et en traitant par distillation le mélange réactionnel caractérisé en ce qu'on réalise la réaction en présence du mélange réactionnel constitué essentiellement d'acroléine, glycérine, acroléineglycérineacétals cycliques et d'eau comme solvant et en l'absence de solvant étranger et, si nécessaire, on ajoute au mélange réactionnel libéré du catalyseur, avant le traitement de distillation, une quantité telle de substance qui augmente le pH, de sorte qu'en dilution au dixième avec de l'eau elle présente un pH compris entre 4,5 et 7.

2. Procédé selon la revendication 1, caractérisé en ce qu'on réalise la réaction à une température comprise entre 0 et 50° C, notamment entre 10 et 30° C.

3. Procédé selon les revendications 1 ou 2, caractérisé en ce qu'on utilise comme catalyseur des échangeurs d'ions organiques ou minéraux fortement acides sous forme de H, notamment des échangeurs ioniques contenant des groupes sulfonates.

4. Procédé selon l'une ou plusieurs des revendications 1 à 3, caractérisé en ce qu'on utilise de l'acroléine et de la glycérine en proportion molaire supérieure à 1 pour 1 jusqu'à 5 pour 1 notamment 1,5 à 2,5 pour 1.

5. Procédé selon l'une ou plusieurs des revendications 1 à 4, caractérisé en ce qu'on utilise comme solvant un mélange réactionnel qu'on obtient par réaction d'acroléine avec la glycérine en proportion molaire de 1,5 à 2,5 et de 40 à 50 % en poids du mélange d'acroléineglycérineacétals cycliques.

6. Procédé selon l'une ou plusieurs des revendications 1 à 5, caractérisé en ce qu'on utilise comme substance augmentant le pH une solution tampon avec un pH compris dans l'intervalle de 5 à 7, de préférence de 5,5 à 6,5.

7. Procédé selon la revendication 6, caractérisé en ce qu'on ajoute au mélange réactionnel une quantité de solution tampon telle que par dilution par dix dans l'eau on obtienne un pH compris entre 5,5 et 7.

8. Procédé selon l'une ou plusieurs des revendications 1 à 7, caractérisé en ce qu'il fonctionne en continu, en injectant en continu l'acroléine et la glycérine en proportion molaire souhaitée en continu au mélange réactionnel servant de solvant, et on fait réagir en présence du catalyseur acide solide, et on prélève en continu la quantité de mélange réactionnel correspondant à la quantité ajoutée et on l'envoie au traitement.
